Europäisches Patentamt

European Patent Office

Office européen des brevets

(19)

(11) Veröffentlichungsnummer : **0 019 608
B1**

(12)   **· EUROPÄISCHE PATENTSCHRIFT**

(45) Veröffentlichungstag der Patentschrift :
20.02.85

(51) Int. Cl.⁴ : **A 61 N   1/32**

(21) Anmeldenummer : **80890049.2**

(22) Anmeldetag : **02.05.80**

---

(54) **Massagegerät.**

---

(30) Priorität : **03.05.79 AT 3336/79**

(43) Veröffentlichungstag der Anmeldung :
**26.11.80 Patentblatt 80/24**

(45) Bekanntmachung des Hinweises auf die Patenterteilung : **20.02.85 Patentblatt 85/08**

(84) Benannte Vertragsstaaten :
**AT BE CH DE FR GB IT LI LU NL SE**

(56) Entgegenhaltungen :
**DE-C-   292 570
FR-A-   659 789
FR-A-   743 153
GB-A-   275 360
US-A- 2 553 873**

(73) Patentinhaber : **Grander, Johann
Haus Ingeborg Nr. 370
A-6373 Jochberg (AT)**

(72) Erfinder : **Grander, Johann
Haus Ingeborg Nr. 370
A-6373 Jochberg (AT)**

(74) Vertreter : **Binder, Otto, Dipl.-Ing.
Stallburggasse 2
A-1010 Wien (AT)**

EP 0 019 608 B1

## Beschreibung

Die Erfindung betrifft ein Massagegerät mit Massagerollen und mit einem aus einem Permanentmagneten und mit einem mit Wicklungen bestückten Rotor bestehenden Stromerzeuger, dessen Antrieb mittels eines Antriebsritzels und einer Antriebswelle von der Drehbewegung einer des Massagerollen abgeleitet ist, die isoliert gelagert und über das Ritzel und die Antriebswelle mit den Gegenpolen der Wicklungen des Stromerzeugers leitend verbunden ist, während die Massepole dieser Wicklungen über einen auf der Antriebswelle isoliert gelagerten Schleifring und einen ersten Teil eines Stromabnehmers mit der mit der anderen Massagerolle verbundenen Masse verbunden sind und zwecks Erzeugung taktweiser Kurzschlußimpulse bei Drehung der Antriebswelle ein zweiter, mit dem ersten leitend verbundener Teil des Stromabnehmers mit einem auf der Antriebswelle angeordneten und mit diesem verbundenen Kontaktglied periodisch zusammenwirkt.

Ein solches Gerät ist bereits aus der FR-A-659 789 bekannt geworden.

Dieses vorbekannt Massagegerät ist allerdings aus einer Vielzahl filigraner und kompliziert zu montierender Einzelteile zusammengesetzt, wodurch dessen Herstellung kostspielig ist.

Sofern man nicht das gesamte Gehäuse stromleitend ausbilden will, bedarf das vorbekannte Gerät eines zeitaufwendig zu befestigenden und raumaufwendig ausragenden Bügels als Halter des Stromabnehmers.

Am oberen Ende der Antriebswelle anzuordnende Scheiben, von denen eine isoliert befestigt werden muß, sind in diesem freien Endbereich der Antriebswelle sehr gefährdet sowie vergleichsweise schwierig zu befestigen und in ihrer Sollstellung zu sichern, wobei naturgemäß die Gefahr auftritt, daß infolge ihrer unmittelbaren Nachbarschaft schon geringe Lageveränderungen zu Betriebsstörungen führen.

Aufgabe vorliegender Erfindung ist es, ein Massagegerät der eingangs bezeichneten Gattung einfacher und robuster als bisher, sowie dementsprechend Störungsunempfindlicher und leichtgewichtiger zu gestalten.

Erfindungsgemäß ist die in ihrem Kern aus leitendem Material bestehende Antriebswelle bereichsweise von einem aus isolierendem Material bestehenden Mantel umhüllt, der sowohl den Rotor als auch den Schleifring trägt, und dieser Mantel und die Antriebswelle sind von einem aus leitendem Material bestehenden Stift als mit dem zweiten Teil des Stromabnehmers periodisch zusammenwirkendes Kontaktglied durchsetzt.

Dank dieser Ausbildung können alle wesentlichen Organe des Massagegerätes unschwer innerhalb des Permanentmagneten, und zwar vorteilhafterweise in unmittelbarer Nähe der Wicklungen angeordnet werden ; dadurch werden die Leitungswege minimal.

Der die Antriebswelle bereichsweise umhüllende Mantel Erfüllt zweckdienlicherweise mehrerlei Funktionen : Er dient nicht nur als gemeinsamer Träger der Wicklungen, der Massepole und des schleifringes, sondern er bettet auch das Kontaktglied, nämlich den Bolzen, der die Antriebswelle quer durchsetzt, so zuverlässig ein, daß von diesem Element keinerlei Störungen ausgehen können, weil es voll umhüllt, dadurch solide gelagert und beschädigungssicher abgeschirmt ist. Die Isolierung aller funktionswesentlicher Bestandteile sowie die Fixierung der Soll-Lage dieser Bestandteile in bezug zueinander ist allein durch diesen Mantel ebenso zuverlässig gewährleistet wie die solide Befestigung und Halterung dieser Bestandteile innerhalb des vom Permanentmagneten umschlossenen Raumes.

Ein Ausführungsbeispiel des Erfindungsgegenstandes sowie zwei Varianten einer stufenlosen Intensitätsregelung sind in den Zeichnungen veranschaulicht, und zwar zeigt die

Figur 1 einen Längsschnitt durch das Gerät mit einer Ansicht des oberen Teiles, die

Figuren 2 und 3 zeigen die erste Variante der Regelvor richtung in einer Ansicht bzw. in einem Schnitt nach III-III der Fig. 2 und die

Figur 4 zeigt die zweite Variante in einer schematischen Darstellung.

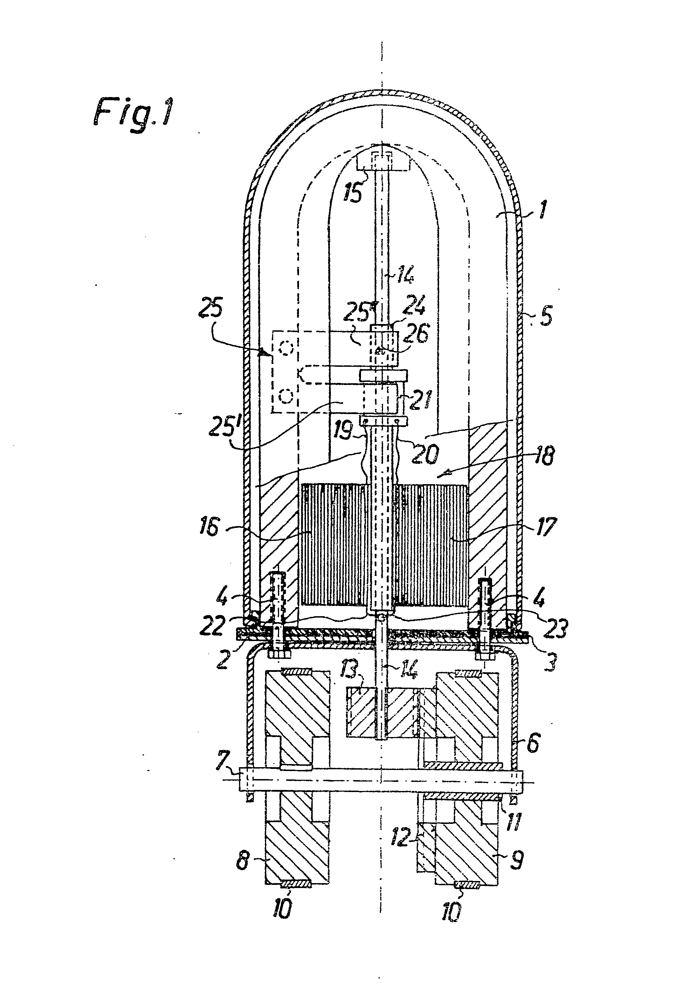

Das in Fig. 1 dargestellte Gerät besteht im wesentlichen aus dem Permanentmagneten 1, der auf einer Basis 2 unter Zwischenschaltung einer isolierenden Zwischenlage 3 mittels der Schrauben 4 befestigt und von einer aus isolierendem Material, z. B. Kunststoff, bestehenden, bedarfsweise abnehmbaren Hülle 5 eingeschlossen ist.

Die Schrauben 4 dienen gleichzeitig auch zur Befestigung eines Tragbügels 6, der die Achse 7 lagert, auf der die beiden aus leitendem Material bestehenden Massagerollen 8 und 9 koaxial befestigt sind. Diese Massagerollen 8 und 9 können mit vorzugsweise ebenfalls aus leitendem Material bestehenden Laufringen 10 bestückt sein, um die für das Abrollen auf der menschlichen Haut ausreichende Griffigkeit zu besitzen. Die in der Zeichnung rechte Massagerolle 9 lagert durch eine Hülse 11 isoliert auf der Achse 7 und besitzt an der der anderen Laufrolle 8 zugewendeten Seite einen Zahnkranz 12, der mit einem Antriebsritzel 13 der Antriebswelle 14 kämmt.

Von dem am unteren Ende dieser Antriebswelle 14 befestigten Antriebsritzel 13 erstreckt sich diese aus Metall od. dgl. leitendem und widerstandsfähigen Material bestehende Antriebswelle 14 durchgehend zum oberen, aus isolierendem Material bestehenden Lager 15, das am Permanentmagneten 1 befestigt ist. Die Antriebswelle 14 trägt zunächst den aus zwei einander gegenüberliegenden, einfachen einzelnen Wicklungen 16 und 17 bestehenden Rotor 18, deren Massepole 19 und 20 an einen Schleifring 21 angeschlossen und dessen Gegenpole 22 und 23 über einen die Antriebswelle 14 durchsetzenden Stift mit dieser Antriebswelle verbunden sind.

Sowohl der Schleifring 21 als auch der Rotor 18 lagern auf einem isolierenden Mantel 24 der Antriebswelle 14.

Dem Schleifring 21 ist ein Teil 25′ eines Stromabnehmers 25 zugeordnet ; der Stromabnehmer ist am Permanentmagneten 1, also an der Masse des Gerätes, leitend befestigt.

Der andere Teil 25″ des Stromabnehmers 25 arbeitet mit den aus dem Mantel 24 zutagetretenden, einander gegenüberliegenden Enden eines Stiftes 26 zusammen, der den Mantel 24 und die Antriebswelle 14 durchsetzt und demnach mit der letzteren leitend verbunden ist.

Die Funktion des Gerätes ergibt sich wie folgt :

Das Gerät wird mit einer Hand an der Hülle 5 erfaßt und die Massagerollen 8 und 9 werden an der zu massierenden Stelle des menschlichen Körpers auf der Haut abgerollt. Die Massagerolle 9 überträgt die ihr dadurch erteilte Drehbewegung über das Antriebsritzel 13 aud die Antriebswelle 14, die ihrerseits den Rotor 18 in Drehung versetzt, wodurch in den Wicklungen 16 und 17 Strom erzeugt wird. Über die Massepole 19, 20 gelangt dieser Strom einerseits zum Schleifring 21 und zum Stromabnehmer 25, weiters über die Masse, den Permanentmagneten 1, und über die Schrauben 4, den Tragbügel 6, die Achse 7 zur Massagerolle 8. Über die Gegenpole 22, 23 der Wicklungen 16, 17 wird anderseits der Antriebswelle 14 zugeführt und gelangt über das Antriebsritzel 13 und den Zahnkranz 12 zur Massagerolle 9.

Der Stromkreis schließt sich zwischen den Massagerollen 8 und 9 über die menschliche Haut, auf der diese Rollen laufen.

Immer dann, wenn der Teil 25″ des Stromabnehmers 25 mit einem der beiden Enden des die Antriebswelle 14 durchsetzenden Stiftes 26 in Kontakt kommt wird der zwischen den Massepolen 19, 20 und den Gegenpolen 22, 23 der Wicklungen 16, 17 fließende Strom über den Stromabnehmer 25 kurzgeschlossen und es ergeben sich taktweise Kurzschlußimpulse hoher Spannung, in einer Frequenz, die der Abrollgeschwindigkeit proportional ist. Diese Impulse übertragen sich in wünschenswerterweise auf die Haut des zu massierenden Körperteiles und ergeben eine für vielerlei Leiden wertvolle Heilwirkung. Bei Benutzung eines Stromerzeugers mit geeigneter Sinuskurven-Charakteristik baut das Gerät eine Spannung von etwa ± 500 V auf, die beim Maximum des Sinus — Abstand von Spitze zu Spitze — einem Spannungsimpuls von 1000 V entspricht. Die Stromstärke ist minimal und beträgt 1,5 bis 5,0 Milliampere.

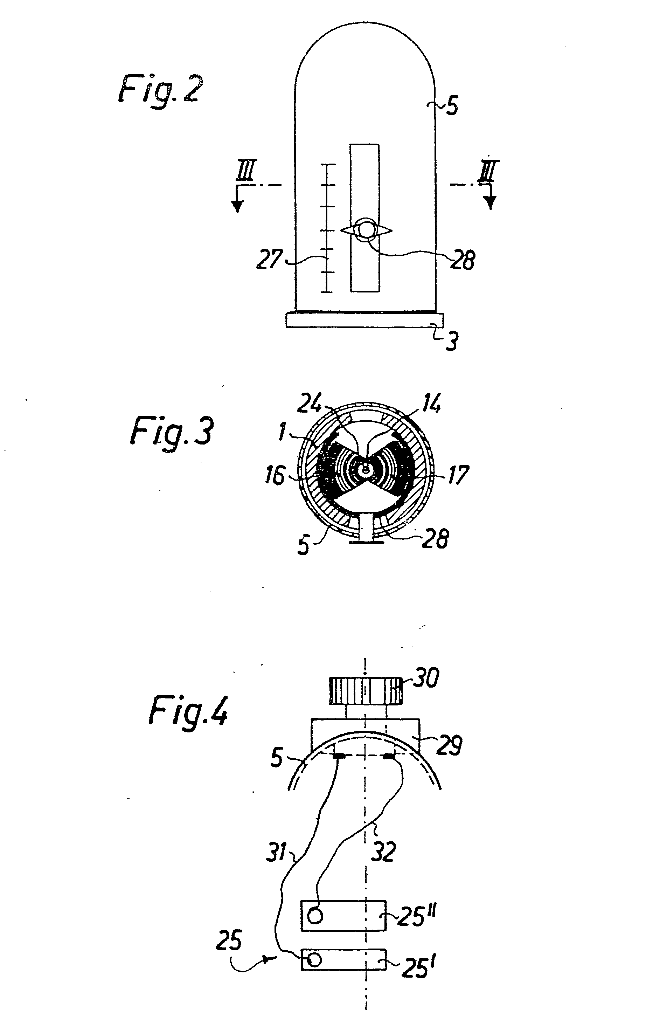

Zur Regelung der Intensität der Impulse kann gemäß Fig. 2 und 3 ein längs des Magnetfeldes des Permanentmagneten 1 verschiebbarer, den Rotor 18 zumindest teilweise umschließender und das Magnetfeld örtlich überbrückender, einer Skala 27 zugeordneter Schieber 28 vorgesehen sein, der die Größe des Magnetfeldes variiert und dadurch auf die Intensität der Kurzschlußimpulse Einfluß nimmt.

Es kann aber anstelle eines solchen Schiebers auch ein Regelwiderstand 29 mit einem Einstellgriff 30 an der Hülle 5 angeordnet werden. Dieser Regelwiderstand 29 ist dann vorteilhaft mit Leitungen 31 und 32 mit den Teilen 25′ und 25″ zu verbinden, die bei dieser Ausführungsform voneinander gesondert am Permanentmagneten 1 als Stromabnehmer 25 am Permanentmagneten anzubringen sind.

Dieser Permanentmagnet kann im Rahmen der Erfindung als Hufeisen, Glocken- oder Haubenmagnet ausgebildet sein, er kann aber auch als magnetisches System ausgebildet werden, so etwa mit im Kopfbereich angeordneten Plattenmagneten und Weicheisenteilen, die sich über den Bereich des Rotors erstrekken und diesen beidseits einschließen.

## Anspruch

Massagegerät mit Massagerollen (8, 9) und mit einem aus einem Permanentmagneten (1) und mit einem mit Wicklungen (16, 17) bestückten Rotor (18) bestehenden Stromerzeuger, dessen Antrieb mittels eines Antriebsritzels (13) und einer Antriebswelle (14) von der Drehbewegung einer (9) der Massagerollen (8, 9) abgeleitet ist, die isoliert gelagert und über das Ritzel (13) und die Antriebswelle (14) mit den Gegenpolen (22, 23) der Wicklungen (16, 17) des Stromerzeugers leitend verbunden ist, während die Massepole (19, 20) dieser Wicklungen (16, 17) über einen auf der Antriebswelle (14) isoliert gelagerten Schleifring (21) und einen ersten Teil (25′) eines Stromabnehmers (25) mit der mit der anderen Massagerolle (8) verbundenen Masse verbunden sind und zwecks Erzeugung taktweiser kurzschlußimpulse bei Drehung der Antriebswelle (14) ein zweiter, mit dem ersten leitend verbundener Teil (25″) des Stromabnehmers (25) mit einem auf der Antriebswelle (14) angeordneten und mit diesem verbundenen Kontaktglied (26) periodisch zusammenwirkt, dadurch gekennzeichnet, daß die in ihrem Kern aus leitendem Material bestehende Antriebswelle (14) bereichsweise von einem aus isolierendem Material bestehenden Mantel (24) umhüllt ist, der sowohl den Rotor (18) als auch den Schleifring (21) trägt, und daß dieser Mantel (24) und die Antriebswelle (14) von einem aus leitendem Material bestehenden Stift (26) als mit dem zweiten Teil (25″) des Stromabnehmers (25) periodisch zusammenwirkendes Kontaktglied durchsetzt sind.

## Claim

A massage apparatus having massage rollers (8, 9) and a current generator which consists of a permanent magnet (1) and [with]* a rotor (18) provided with windings (16, 17) and whose drive is derived by means of a drive pinion (13) and a drive shaft (14) from the angular movement of one (9) of the massage rollers (8, 9), which is mounted in an insulated manner and is conductively connected to the antipoles (22, 23) of the windings (16, 17) of the current generator by way of the pinion (13) and the drive shaft (14), while the earth poles (19, 20) of the said windings (16, 17) are connected by way of a slip ring (21) mounted in an insulated manner on the drive shaft (14) and a first part (25') of a current collector (25) to earth, to which the other massage roller (8) is connected, and in order to generate timed short-circuit pulses during the rotation of the drive shaft (14) a second part (25") of the current collector (25) conductively connected to the first part co-operates periodically with a contact member (26) mounted on the drive shaft (14) and connected thereto, characterised in that the drive shaft (14) having a core of conducting material is surrounded in given areas by a sleeve (24) which consists of insulating material and which carries both the rotor (18) and the slip ring (21), and a pin (26) consisting of conducting material and acting as a contact member co-operating periodically with the second part (25") of the current collector (25) passes through the said sleeve (24) and the drive shaft (14).

**Revendication**

Appareil de massage avec des rouleaux de massage (8, 9) et avec un générateur de courant constitué par un aimant permanent (1) et par un rotor (18) équipé d'enroulements (16, 17) dont l'entraînement est dérivé au moyen d'un pignon (13) et d'un arbre d'entraînement (14) du mouvement de rotation de l'un (9) des rouleaux de massage (8, 9), qui est monté de façon isolée et est relié de façon conductrice par l'intermédiaire du pignon (13) et de l'arbre d'entraînement (14) aux contre-pôles (22, 23) des enroulements (16, 17) du générateur de courant, alors que les pôles de masse (19, 20) de ces enroulements (16, 17) sont reliés par l'intermédiaire d'une bague de frottement (21) montée de façon isolée sur l'arbre d'entraînement (14) et d'une première partie (25') d'un collecteur de courant (25) avec la masse reliée à l'autre rouleau de massage (8), et un second organe de contact (26) monté sur l'arbre d'entraînement (14) et relié à celui-ci qui coopère périodiquement, en vue de produire des impulsions de court-circuit rythmées pendant la rotation de l'arbre d'entraînement (14), avec la première partie reliée de façon conductrice (25") du collecteur de courant (25), caractérisé en ce que l'arbre d'entraînement (14) constitué dans sa partie de cœur en un matériau conducteur est partiellement entouré par un manchon (24) en un matériau isolant, qui supporte aussi bien le rotor (18) que la bague de frottement (21), et en ce que ce manchon (24) et l'arbre d'entraînement (14) sont traversés par une tige (26) constituée en un matériau conducteur et formant l'organe de contact qui coopère périodiquement avec la seconde partie (25") du collecteur de courant (25).

Fig.1

Fig.2

5

III          III

27          28

3

Fig.3

24          14
1
16          17
5          28

Fig.4

30

29
5

31          32

25''

25          25'